(19) Europäisches Patentamt
European Patent Office
Office européen des brevets

(11) **EP 3 806 025 A1**

(12) **EUROPEAN PATENT APPLICATION**
published in accordance with Art. 153(4) EPC

(43) Date of publication:
**14.04.2021  Bulletin 2021/15**

(51) Int Cl.:
***G06T 3/40*** *(2006.01)*

(21) Application number: **19807475.9**

(86) International application number:
**PCT/ES2019/070343**

(22) Date of filing: **23.05.2019**

(87) International publication number:
**WO 2019/224415 (28.11.2019 Gazette 2019/48)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority:  **24.05.2018  ES 201830502**

(71) Applicant: **Servicio Andaluz de Salud**
**41071 Sevilla (ES)**

(72) Inventors:
• **DE MENA GARCÍA, David**
  **41071 Sevilla (ES)**
• **SÁNCHEZ GARCÍA, Alfonso**
  **41071 Sevilla (ES)**
• **GARCÍA ROJO, Marcial**
  **41071 Sevilla (ES)**

(74) Representative: **ABG Intellectual Property Law, S.L.**
**Avenida de Burgos, 16D**
**Edificio Euromor**
**28036 Madrid (ES)**

(54) **COMPUTER-IMPLEMENTED METHOD FOR CONVERTING A HIGH-RESOLUTION DIGITAL IMAGE INTO A DICOM PYRAMID IMAGE**

(57)    The invention discloses a method for converting a high-resolution digital image into a DICOM pyramidal image. More specifically, the method converts an image having a fixed width (W) and a fixed length (L) into a DICOM pyramidal image comprising a desired number of DICOM instances ($n_i$) each formed by respective pluralities of DICOM frames. The method comprises the following steps: selecting the desired number of DICOM instances ($n_i$); calculating the DICOM frame width ($W_{frame}$) and length ($L_{frame}$) by means of the following formulas: $W_{frame} = \frac{W}{2^{n_i-1}}$ and $L_{frame} = \frac{L}{2^{n_i-1}}$;

and building the DICOM pyramidal image based on said number of DICOM instances ($n_i$) and said DICOM frame width ($W_{frame}$) and length ($L_{frame}$).

FIG.4

EP 3 806 025 A1

## Description

## OBJECT OF THE INVENTION

The object of the present invention is a new computer implemented method particularly designed to convert a high-resolution image, for example an image obtained after scanning or digitizing a histological or cytological preparation in Anatomic Pathology, from an origin format to a DICOM format.

## BACKGROUND ART

### Digitization technology

In the field of medicine, particularly in Anatomic Pathology, the need to make histological and cytological preparations for a subsequent analysis by a clinician is known. A number of devices for digitizing said preparations have recently appeared. Digitization devices, known as scanners, obtain a high-resolution digital image of the preparation, which is thus available for analysis any place and time without the need for the clinician to have physical access to the preparation.

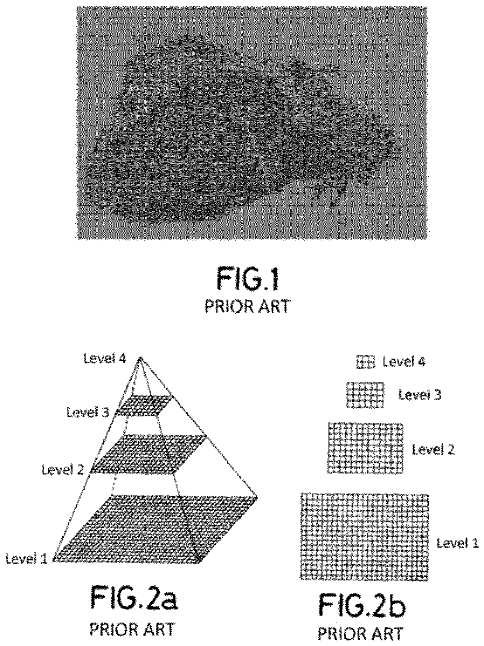

The digitization method carried out by these scanners for obtaining a digital high-resolution image of the preparations comprises taking a mosaic of photographs, normally square-shaped photographs, of small fragments of the surface of the preparation known as "cells". The scanners comprise a digital camera with a 20x to 40x zoom lens, mounted on a structure holding the camera over the preparation. A parallel relative movement between the camera and the preparation is generated while the camera takes plurality of pictures of the preparation from above. The plurality of pictures are taken in positions separated by a small distance, such as microns. Scanners are usually designed for providing an overlap band between each pair of adjacent photographs or cells. That is, every individual photograph overlaps a predetermined distance with the photographs located immediately below, above, and to either side. Subsequently, every photograph is placed in the correct position in the image, and a software generating the complete image operates with the images such that said overlap bands are not visible for a clinician analyzing the image. Fig. 1 shows an exemplary image of a digitized histological preparation according with this method.

A drawback of this technology is related with the huge size of the complete digitized high-resolution image. Indeed, a high-resolution image of this type may contain several thousands of individual photographs. The size of such a high-resolution image may therefore range between several hundreds of megabytes to about tens of gigabytes. Obviously, managing and viewing an image having this size is very complicated.

In order to solve this drawback, the use of image composition method known as "pyramidal" for visualizing these high-resolution images is known. This visualization method comprises creating a number of lower resolution images usually known as "levels". Usually, the size of the image making up each level is half the size of the image making up the level immediately below. The image of each level is also formed by a plurality of cells. Therefore, starting from the complete image having the highest resolution that makes up the lowermost level at the base of the pyramid, successive levels having gradually lower resolutions are created, until the uppermost level having the lowest resolution that makes up the top of the pyramid. Fig. 2a schematically shows the pyramidal structure in a case where the original image is level 1 of the pyramid and the top of the pyramid is level 4. On the other hand, Fig. 2b shows the image corresponding to each level making up the pyramid of Fig. 2a.

During visualization of the image, the user initially views the uppermost level having the lowest resolution. When the user desires to view a particular area of the image with a greater resolution, the visualization software accesses to the immediately lower level. This process takes place as many times as necessary until the user views the highest resolution image at the base of the pyramid. This method effectively limits the size of the image files the software must manage at any given moment. This image visualization method is very similar to the method employed Google Maps®, a tool provided by Google®.

Therefore, the current digitalization technology mainly provides three elements for defining an image: complete image, level, and cell. Each complete image comprises several levels, and every level in turn comprises a plurality of cells each corresponding to an individual photograph.

### Proprietary formats

Each digitization device manufacturer uses a different proprietary format for storing and visualizing the images. Each proprietary format may have, for example, overlap bands of a different width, a different number of levels, a different cell size, etc. Thus, a digitized image can only be viewed using a specific visualization software provided by the manufacturer of the device used to carry out the digitization.

Consequently, health care services are required to use a specific proprietary technology. Further, the possibility of exchanging images and studying different diagnostic images is seriously hindered. This is obviously a very important drawback.

### DICOM format

DICOM (Digital Imaging and COmmunication in Medicine) is a standard for storing and exchanging medical images enabling the integration of medical imaging devices such as scanners, servers, workstations, printers, network hardware, and picture archiving and communication systems (PACS) from multiple manufacturers. It has been widely adopted by hospitals. Further, supplements 145 and 122 of DICOM define the necessary extensions for storing and visualizing high-resolution images used in Anatomic Pathology.

A simplified entity-relationship defined by DICOM establishes the "patient" as the main node. Each patient may have a number of "studies". Each study may be divided in one or more "series". Each series may comprise several "instances". And, finally, each instance may comprise a single image or an image in a plurality of "frames". For Digital Pathology images, DICOM Supplement 145 defines the use of multiframe images. This structure corresponds to a class denoted as VL Whole Slide Microscopy Image, (WSI). The lowest level in this structure, the frame, is simply an image separated a constant distance from a previous image and a subsequent image. Importantly, DICOM format does not define an overlap band between adjacent frames. The frames are placed side-by-side without any overlap.

So the DICOM format defines three elements essentially equivalent to those employed in any of the proprietary formats defined by the digitization device manufacturers: series, instance, and frame. Every series comprises several instances, and in turn every instance comprises a plurality of frames.

Therefore, every series defined by DICOM may be equivalent to an image according to a proprietary format, every instance defined by DICOM may be equivalent to a level defined by a proprietary format, and every frame defined by DICOM may be equivalent to a cell defined by a proprietary format.

| Proprietary format | | DICOM format |
|---|---|---|
| Image | ------------→ | Series |
| Level | ------------→ | Instance |
| Cell | ------------→ | Frame |

**Conversion between proprietary format and DICOM format**

In view of the above, methods for converting the high-resolution images used in Anatomic Pathology from a proprietary format into DICOM are necessary. The use of said methods allows for applying DICOM WSI standard in Anatomic Pathology, enabling the exchange and visualization of images generated by digitization devices of different manufacturers.

Nowadays, a number of methods for converting a proprietary format image into a DICOM format image are known. These methods basically take the complete, stitched image formed by the plurality of photographs and re-cut it into newly defined fragments defined by the DICOM standard.

However, the DICOM standard does not define the size of each of the individual frames belonging to each instance. In view of this, the first step in current conversion methods is selecting a size for the frames. This selection is usually arbitrary. For example, most current conversion methods merely select the same size cells in the original proprietary format had. In any case, selecting an arbitrary frame size is disadvantageous in that the edge of the last frame in a row or column of the image that corresponds to the lowermost level in DICOM format does not match the edge of the image corresponding to the lowermost level in the proprietary format. In this respect, note that the total size of the complete image, that is, the number of pixels containing image information, is necessarily the same irrespective of the digitization format. Consequently, the DICOM format image generated by any of these known conversion methods has an empty horizontal band at the right edge of the image and an empty vertical band at the lower edge of the image. These bands are usually represented in black or white color.

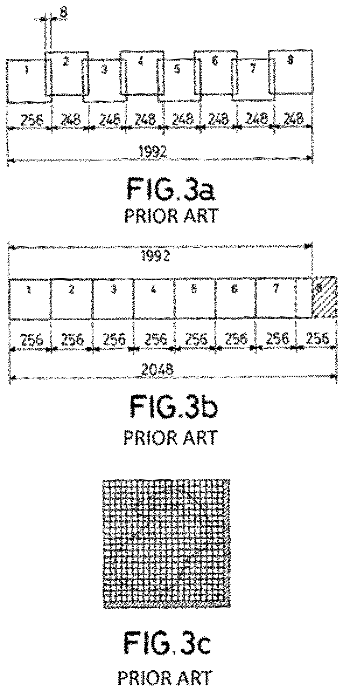

Figs. 3a-3c schematically show this problem in a unidimensional example for ease of understanding. Fig. 3a shows a particular row of an Anatomic Pathology image formed only by 8 cells having a length of 256 pixels each. Let's assume that each cell overlaps the adjacent cell an 8 pixel wide band, then the total length of the row is 1992 pixels.

Now, as disclosed above, present conversion methods take the complete, stitched image and re-cut it according to the desired DICOM frame size. Since the DICOM standard does not define a particular size for the frames, known conversion methods merely use the same size cells had in the original proprietary format. Therefore, in this example 256 pixel wide frames are employed. However, the DICOM does not define an overlap between frames. Consequently, the total length of a row formed by 256 pixel long frames in DICOM format does not match the length of the row formed by 256 pixel long cells in proprietary format. Specifically, the total length of the image in DICOM format is 2048 pixels, but the original complete image, that is, those pixels containing image information is only 1992 pixels long. Therefore, the 56 rightmost pixels of the rightmost located frame do not contain any image information. The usual method is assigning these pixels a value of 0 (black) or 256 (white).

Fig. 3c shows the result of the above conversion methods in a two dimensional image. In this example, horizontal and vertical black bands appear at the rightmost and lowermost sides of the image. This occurs in each of the instances of the generated DICOM format image.

**DESCRIPTION OF THE INVENTION**

The present invention solves the drawbacks described above by means of a new computer implemented method for converting the digital image from the proprietary format into the DICOM format where a frame size matching the width and length of the image can be selected. More specifically, the present method prompts the user to select the desired number of instances and, based on said number of instances and the fixed size of the image, calculates an optimal frame size. Thus, the need to provide a black or white band adjacent the generated DICOM format image is eliminated.

The present invention is directed to a computer implemented method for converting a high-resolution digital image into a DICOM pyramidal image. The method converts an image having a fixed width and a fixed length into a DICOM pyramidal image comprising a desired number of DICOM instances each formed by respective pluralities of DICOM frames. The method comprises the following steps:

1) Selecting the desired number of DICOM instances.

2) Calculating the DICOM frame width and length by means of the following formulas:

$$W_{frame} = \frac{W}{2^{n_i - 1}}$$

$$L_{frame} = \frac{L}{2^{n_i - 1}}$$

where:

| | |
|---|---|
| W | is the fixed total width of the image. |
| L | is the fixed total length of the image. |
| $W_{frame}$ | is the width of each DICOM frame. |
| $L_{frame}$ | is the length of each DICOM frame. |
| $n_i$ | is the number of DICOM instances. |

3) Building the DICOM pyramidal image based on said number of DICOM instances and said DICOM frame width and length.

While the method of the invention is particularly designed to be executed in a computer, the invention further encompasses a computer program comprising instructions capable of causing a computer to carry out the method of the invention. The program may be in the form of font code, object code, or an intermediate code between font code and object code, such as a partially compiled form. Generally speaking, the program may be in any computer readable form capable of causing a computer to carry out the method of the invention.

The invention further encompasses a computer program stored in a storing means. The storing means could be of any kind, such as e.g. a ROM, a CD-ROM, a semiconductor ROM, a hard disk, etc. Alternatively, the storing means could be an ASIC adapted to store and execute the method of the invention. In any case, generally speaking the storing means includes any kind of means capable of storing the list of instructions making up the computer program.

The invention also encompasses a computer program supported in a carrier. In this context, when the program is incorporated in a signal that can be transported by cable or by any other means, such as an electrical or optical signal, the carrier takes the form of said cable or other means.

## BRIEF DESCRIPTION OF THE DRAWINGS

Fig. 1 shows an example of an Anatomic Pathology digitized by means of a plurality of small size photographs.

Figs. 2a and 2b schematically show the pyramidal structure used for visualizing the digitized images.

Figs. 3a, 3b and 3c schematically show the present drawbacks in connection with the the conversion of a digitized image from a proprietary forma into a DICOM format.

Fig. 4 shows a schematic view of the digitized image to be converted into DICOM format.

Figs. 5a-5d shows schematic views of the respective DICOM instances of the DICOM format image generated by the method of the invention.

## PREFERRED EMBODIMENT OF THE INVENTION

An example illustrating the present invention is now disclosed with reference to the drawings.

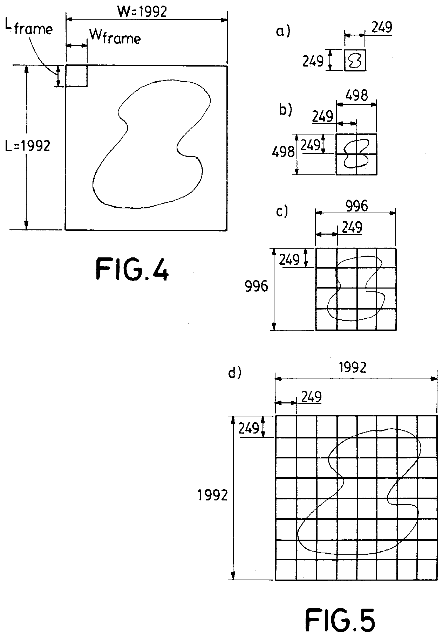

In this example, the image to be converted into a DICOM format image is a bidimensional version of the monodimensional example shown in Figs. 3a-3c. That is, the image in proprietary format is formed by 64 cells (8 x 8), where each cell has a width and length of 256 pixels. The overlap band has a width of 8 pixels. Therefore, the image has a total width (W) of 1992 pixels and a total length (L) of 1992 pixels. Fig. 4 shows the 1992 x 1992 pixel image that is going to be converted into a DICOM format image.

In order to carry out the conversion, the frame size ($W_{frame}$, $L_{frame}$) must be selected. Indeed, the optimal frame size ($W_{frame}$, $L_{frame}$) depends on the number of instances the user desires to divide the image into. Therefore, the present method prompts the user to select the desired number of instances. In the present example, the number of instances is $n_i=4$.

The method then calculates the optimal frame width (Wframe) and frame length (Lframe) by means of the formulas disclosed above:

$$W_{frame} = \frac{W}{2^{n_i-1}} = 249 \text{ pixels}$$

$$L_{frame} = \frac{L}{2^{n_i-1}} = 249 \text{ pixels}$$

Finally, the method builds a DICOM format image comprising 4 instances formed by frames having a size of 249 x 249 pixels. The result is shown in Figs. 5a-5d: Fig. 5a shows the uppermost instance formed by a single 249 x 249 frame; Fig. 5b shows the instance immediately below formed by four 249 x 249 frames; Fig. 5c shows the instance immediately below formed by sixteen 249 x 249 frames; and Fig. 5d shows the lowermost instance formed by sixty four 249 x 249 frames. The size of the lowermost instance is therefore 1992 x 1992, matching exactly the original image. Therefore, there is no need to add a black or white band made of pixels having no information.

## Claims

1. Computer implemented method for converting a high-resolution digital image into a DICOM pyramidal image, the method converting an image having a fixed width (W) and a fixed length (L) into a DICOM pyramidal image comprising a desired number of DICOM instances ($n_i$) each formed by respective pluralities of DICOM frames, comprising the following steps:

   - selecting the desired number of DICOM instances ($n_i$);
   - calculating the DICOM frame width ($W_{frame}$) and length ($L_{frame}$) by means of the following formulas:

   $$W_{frame} = \frac{W}{2^{n_i-1}}$$

   $$L_{frame} = \frac{L}{2^{n_i-1}};$$

   and
   - building the DICOM pyramidal image based on said number of DICOM instances ($n_i$) and said DICOM frame width ($W_{frame}$) and length ($L_{frame}$).

2. Computer program comprising program instructions for making a computer carry out the method according to claim 1.

3. Computer program according to claim 2, stored in storing means.

4. Computer program according to claim 2, supported in a carrier.

**FIG.1**
PRIOR ART

**FIG.2a**
PRIOR ART

**FIG.2b**
PRIOR ART

**FIG.3a**
PRIOR ART

**FIG.3b**
PRIOR ART

**FIG.3c**
PRIOR ART

FIG.4

FIG.5

8

# INTERNATIONAL SEARCH REPORT

International application No.

PCT/ES2019/070343

## A. CLASSIFICATION OF SUBJECT MATTER

*G06T3/40* (2006.01)

According to International Patent Classification (IPC) or to both national classification and IPC

## B. FIELDS SEARCHED

Minimum documentation searched (classification system followed by classification symbols)

G06T

Documentation searched other than minimum documentation to the extent that such documents are included in the fields searched

Electronic data base consulted during the international search (name of data base and, where practicable, search terms used)

EPODOC, INVENES, Internet, WPI

## C. DOCUMENTS CONSIDERED TO BE RELEVANT

| Category* | Citation of document, with indication, where appropriate, of the relevant passages | Relevant to claim No. |
|---|---|---|
| X | NEMA. DICOM Whole Slide Imaging. www.dicom.nema.org, 20/09/2017 [on line][retrieved on 07/08/2019]. Retrieved from Internet <URL: https://web.archive.org/web/20180606032436/ http:/dicom.nema.org/Dicom/DICOMWSI/> Figures 4 and 5, Access patterns, data organization | 1-4 |
| A | Zoom levels. Wiki Open Streetmap, 20/12/2017 [on line][retrieved on 07/08/2019]. Retrievedof Internet <URL: https://web.archive.org/web/20171220163644/ http://wiki.openstreetmap.org/wiki/Zoom_levels> the whole document | 1-4 |
| A | TAKKAT. Crop a big picture into several small size pictures. StackExchange, 22/04/2014 [on line][retrieved on 07/08/2019]. Retrieved of Internet <URL: https://web.archive.org/web/20161126155518/ https:/graphicdesign.stackexchange.com/questions/30008/crop-a-big-picture-into-several-small-size-pictures> Respuesta of Takkat | 1-4 |

☒ Further documents are listed in the continuation of Box C.　　☐ See patent family annex.

| * | Special categories of cited documents: |
|---|---|
| "A" | document defining the general state of the art which is not considered to be of particular relevance. |
| "E" | earlier document but published on or after the international filing date |
| "L" | document which may throw doubts on priority claim(s) or which is cited to establish the publication date of another citation or other special reason (as specified) |
| "O" | document referring to an oral disclosure use, exhibition, or other means. |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other documents , such combination being obvious to a person skilled in the art |
| "&" | document member of the same patent family |

| Date of the actual completion of the international search | Date of mailing of the international search report |
|---|---|
| 08/08/2019 | **(08/08/2019)** |

| Name and mailing address of the ISA/ | Authorized officer |
|---|---|
| OFICINA ESPAÑOLA DE PATENTES Y MARCAS Paseo de la Castellana, 75 - 28071 Madrid (España) Facsimile No.: 91 349 53 04 | F. Diaz Madrigal Telephone No. 91 3493287 |

Form PCT/ISA/210 (second sheet) (January 2015)

**INTERNATIONAL SEARCH REPORT**

| International application No. |
| --- |
| PCT/ES2019/070343 |

| C (continuation). | DOCUMENTS CONSIDERED TO BE RELEVANT | |
| --- | --- | --- |
| Category * | Citation of documents, with indication, where appropriate, of the relevant passages | Relevant to claim No. |
| A | PHOTO ADVANCED. Slice Image into Pieces – GIMP 2.8 Tutorial. Youtube, 23/09/2016 [on line][retrieved the 06/08/2019]. Retrieved from Internet <URL: https://web.archive.org/web/20190808071523/ https://www.youtube.com/watch?v=XNnpvNpPowk> the whole document | 1-4 |

Form PCT/ISA/210 (continuation of second sheet) (January 2015)